# EUROPEAN PATENT SPECIFICATION

(11) **EP 3 320 931 B1**
(45) Date of publication and mention of the grant of the patent: **26.02.2020**
(21) Application number: 16821530.9
(22) Date of filing: 17.05.2016
(51) Int. Cl.: A61M 5/168, A61M 39/10

(54) **SELECTOR**
WÄHLSCHALTER
SÉLECTEUR

(30) Priority: 08.07.2015 KR 20150097269
(43) Date of publication of application: 16.05.2018
(73) Proprietor: Cebika Inc., Uiwang-si, Gyeonggi-do 16006 (KR)
(72) Inventor: CHO, Byoung Chic, Seongnam-si Gyeonggi-do 13582 (KR)
(74) Representative: Hansen, Norbert
(86) International application number: PCT/KR2016/005211
(87) International publication number: WO 2017/007124

(56) References cited:
- KR-A- 20100 027 806
- KR-B1- 100 578 001
- US-A1- 2007 215 637
- US-A1- 2010 057 015
- US-A1- 2013 138 075
- US-A1- 2013 138 075
- US-A1- 2013 178 805
- US-A1- 2013 178 805
- US-A1- 2014 182 591
- US-A1- 2014 182 591
- US-B2- 7 803 133

## Description

### Technical Field

The present invention relates to a selector, and more particularly, to a selector that may interrupt or regulates flows of a fluid, and selectively discharge a preset quantity of the introduced fluid.

### Background Art

The technology of adjusting an amount of a fluid flowing through a pipe to a desired amount and discharging the fluid is used in various fields, but for example, a medicine introduced or infused into the patient in the medical field has to be injected naturally according to its usage, and an infusion amount, an infusion speed, and an infusion time are very important factors.

In particular, a usage of a pain killer used for adjustment of pain of the patient after a surgery is particularly important, and if the usage is not observed, symptoms, such as respiratory depression, an excessive sedative action, and a stomach/intestine problem including nausea-vomiting, a urinary retention, and itching, which are side effects due to the type of the medicine may be caused, and in a severe case, drug addiction may be caused.

In the case of the pain killer, in recent years, a patient controlled analgesia (PCA) method for adjusting pain uniformly has been used instead of intermittently infusing a medicine whenever the patient complains of pain.

The PCA method is a method for restraining pan while continuously infusing a medicine that shows relatively low side-effects and high effects to a small degree, and allowing the patient to additionally directly inject the medicine to release pain due to an abrupt state change.
degree, and allowing the patient to additionally directly inject the medicine to release pain due to an abrupt state change.

In order to adjust basic pain, as described above, it is very important to continuously determine and finely adjust a dose to continuously inject the medicine, and when the adjustment fails, a side-effect of the pain killer is generated.

As an apparatus for adjusting continuous injection of a medicine, a flow rate adjusting apparatus generally used in an infusion solution such as linger adjusts flow rate by applying a pressure to a flexible tube, adjusting the pressure, and changing a cross-sectional area of the tube, and has a simple structure including only two to three components.

However, in the flow rate adjusting apparatus, a disk manipulated by a thumb moves from an upper end to a lower end of a housing while rolling along the housing, it is considerably unnatural to manipulate the disk rolling along the housing with the thumb in a state in which the housing is gripped by the thumb and the middle finger and it is very difficult for an unskilled person to accurately adjust an amount of chemical by rolling the disk. Accordingly, as long as the kind of the medicine is accurately prescribed, it is possible to use the apparatus in injecting the medicine such as a linger liquid relatively conveniently, but it is not only difficult but also dangerous to apply the medicine, such as a pain killer, which causes a serious side effect if the medicine is misused.

Meanwhile, Korean Patent No. 10-1058539 discloses an infusion solution flow rate adjustor that may promptly adjust a prescribed flow rate by setting a manipulation point of a manipulation unit at a ratio of the flow rate measured at a reference point for the prescribed flow rate.

However, in the infusion solution flow rate adjustor according to the related art, a pressure at an input end of an infusion liquid applied to the adjustor according to the amount of the infusion solution residing in an infusion solution storage container is changed, and the flow rate of the infusion solution cannot be adjusted according to the lapse of time due to the change of the pressure.

In addition, Korean Patent Application Publication No. 2007-0030420 discloses an apparatus for determining an amount of injected insulin in a syringe by connecting a plunger, to which a driving motor is connected, to the syringe, in which insulin is accommodated, to electronically control a driving motor.

Because lethal side-effects are caused when an excessive amount of insulin is injected, the danger of the side-effects is reduced by using the apparatus, but the amount of insulin injected by using the apparatus is determined in advance and solves a problem of at which speed the determined amount of insulin is to be injected. Further, because the capacity of the syringe is determined, the apparatus cannot be applied when it is necessary to continuously inject the determined amount of insulin per unit time. Further, because the apparatus requires a driving motor for controlling the plunger and electronic equipment, the apparatus becomes larger and more expensive.
(Patent Document 1) KR 1058539 B1
(Patent Document 2) KR 2007-0030420 A

US 2013/0138075 A1 describes a device for controlling the rate of infusion of fluids during infusion therapy using non-electric infusion devices. Rotation of a flow regulator dial causes an orifice connected to the inlet to modify its position relative to a particular one or more orifices or groove portions, the characteristics of which provide a certain flow rate characteristic. The regulator allows for the infusion pump to infuse at a rate that may be varied during use by the user.

US 2010/0057015 A1 describes a medicinal liquid supply apparatus including a fixed-type medicinal liquid supply volume controller having a plurality of medicinal liquid branch hoses opened and closed by a controller and a medicinal liquid transfer hose always opened, the medicinal liquid branch hoses and the medicinal liquid transfer hose being connected to an inflow part and an outflow part of a medicinal liquid control line thereof; and an arbitrary medicinal liquid supply volume controller connected to a double medicinal liquid hose having a branch tube c01mected to the medicinal liquid transfer hose of the fixed-type medicinal liquid supply volume controller, the arbitrary medicinal liquid supply volume controller having a medicinal liquid storing tube for storing the medicinal liquid transferred through the double medicinal liquid hose to arbitrarily and additionally supply the medicinal liquid by a button body.

### Disclosure

### Technical Problem

Accordingly, the present invention has been made in an effort to solve the above-mentioned problems, and provides a selector that may continuously inject a fluid by adjusting the flow rate of the fluid with a simple manipulation and may be simply manufactured.

### Technical Solution

The invention is defined by the subject-matter of claim 1.

In accordance with an aspect of the present invention, there is provided a selector including a body, an inlet located on one side of the body, an outlet located on an opposite side of the body, a circular upper passage, one side of which communicates with the inlet and which is located on an upper side of the body, a circular lower passage, one side of which communicates with the outlet and which is disposed on a lower side of the upper passage in parallel to the upper passage, a plurality of tubes disposed in parallel along circumferences of the upper passage and the lower passage, having different inner diameters, and lower side of which communicate with the lower passage, and a first sealing member located to be rotatable to cover the upper passage and upper sides of the plurality of tubes, on an upper side of the body and having a communication recess disposed to or not to overlap the upper passage and an upper portion of any one of the plurality of tubes, on a lower surface thereof, wherein the upper passage and the lower passage selectively communicate with the tubes according to a location of the communication recess through rotation of the first sealing member.

It is preferable that the selector further include a control lever located on the upper side of the body and the first sealing member be located on one side of the control lever to be rotated in conjunction with the control lever as the control lever is rotated.

It is preferable that the first sealing member include a plurality of protrusions protruding from an outer peripheral surface of the first sealing member, the control lever include recesses formed on one side of an inner peripheral surface of the control lever and the number of which is the same as that of the protrusions, and the first sealing member be rotated in conjunction with the first sealing member as the control lever is rotated as the plurality of protrusions is engaged with the recesses to be fixed.

It is preferable that the widths of the plurality of protrusions be different, and the widths of the recesses correspond to the widths of the plurality of protrusions.

It is preferable that the selector further include a display part located on one side of the control lever.

It is preferable that the selector further include a second sealing member located on a lower side of the body and covering the tubes and the lower passage.

It is preferable that movement of the tubes in lengthwise directions thereof be restricted by vertically locating the tubes between the first sealing member and the second sealing member.

It is preferable that a fixing boss be further provided on one side of an inner peripheral surface of the control lever, the body include a plurality of fixing recesses formed at locations corresponding to the tubes along an upper circumference of the body, and the fixing boss be inserted into any one of the plurality of fixing recesses as the control lever is rotated.

### Advantageous Effects

As described above, according to the selector of the present invention, because the fluid introduced may be accurately discharged at a predetermined flow rate according to the set inner diameters of the tubes through a simple manipulation, the selector may be conveniently used and an accurate flow rate may be consistently adjusted, and the selector may be conveniently manufactured without using an adhesive.

### Description of the Invention

Fig. 1 is a perspective view of a selector according to an embodiment of the present invention;
Fig. 2 is an exploded perspective view of the selector according to the embodiment of the present invention;
Fig. 3 illustrates perspective views of a body 100 that is a configuration of the selector according to the embodiment of the present invention, and are views that are taken from the upper side (left) and the lower side (right);
Fig. 4 illustrates perspective views of a control lever 400 that is a configuration of the selector according to the embodiment of the present invention, and are views that are taken from the upper side (left) and the lower side (right);
Fig. 5 illustrates perspective views of a first sealing member 300 that is a configuration of the selector according to the embodiment of the present invention, and are views that are taken from the upper side (left) and the lower side (right);
Fig. 6 is a plan view of the body 100 that is a configuration of the selector according to the embodiment of the present invention, and illustrates flows of a fluid according to a location of a communication recess;
Fig. 7 is a sectional view of the body 100 that is a configuration of the selector according to the embodiment of the present invention, which is taken along line A-A' of Fig. 6, and illustrates flows of a fluid according to a location of a communication recess;
Fig. 8 is a plan view of the body 100 that is a configuration of the selector according to the embodiment of the present invention, and illustrates flows of a fluid according to a location of a communication recess;
Fig. 9 is a sectional view of the body 100 that is a configuration of the selector according to the embodiment of the present invention, which is taken along line B-B' of Fig. 6, and illustrates flows of a fluid according to a location of a communication recess;
Fig. 10 is a bottom view of the body 100 that is a configuration of the selector according to the embodiment of the present invention, and illustrates flows of a fluid according to a location of a communication recess; and
Fig. 11 is a perspective view of the selector according to the present invention, and illustrates another embodiment of the control lever 400'.

### Best Mode

The objectives, features, and other advantages of the present invention will be more apparent by describing exemplary embodiments of the present invention in detail with reference to the accompanying drawings. It should be noted that the drawings are not to precise scale and may be exaggerated in thickness of lines or size of components for descriptive convenience and clarity. In addition, terms used herein are defined by taking functions of the present invention into account and can be changed according to user or operator's custom or intention. Therefore, definition of the terms should be made according to the overall disclosure set forth herein.

Further, the embodiments are provided exemplarily for description of the present invention, and are not intended to limit the technical scope of the present invention.

The elements constituting the selector according to the present invention may be integrally used or separately used as occasion demands. Further, some elements may be omitted according to the usage of the apparatus.

Hereinafter, a selector according to an embodiment of the present invention will be described with reference to the accompanying drawings.

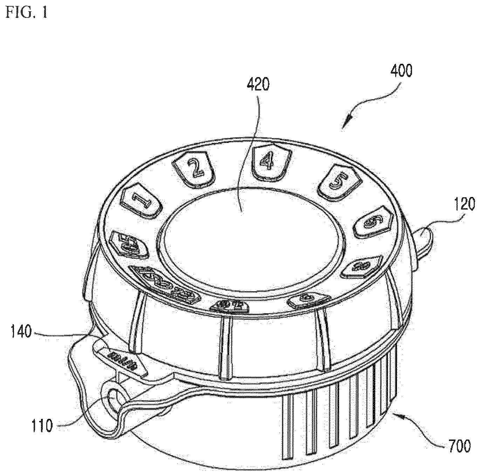

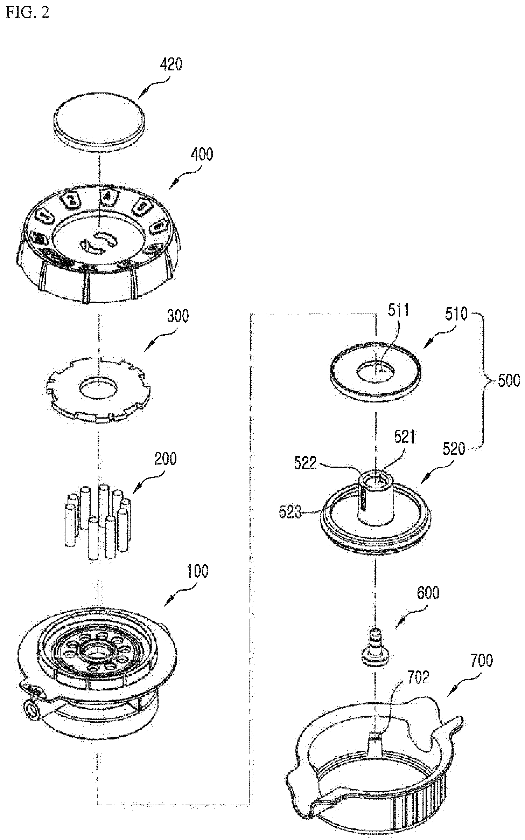

As illustrated in Figs. 1 and 2, the selector according to the embodiment of the present invention may include a body 100, a tube 200, a sealing member (first and second sealing members 300 and 500), a control lever 400, and a casing 700.

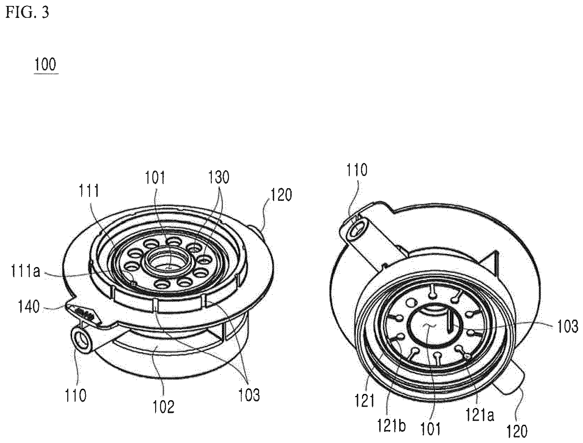

As illustrated in Fig. 3, the body 100 may include an inlet 110, an outlet 120, an upper passage 111, a lower passage 121, and an accommodation part 130.

The inlet 110 is located on one side of the body 100, and a fluid is introduced through the inlet 110.

The outlet 120 is located on the other side of the body 100, and the fluid introduced through the inlet 110 is discharged through the outlet 120 through flows thereof due to operations of the elements, which will be described below.

The locations of the inlet 110 and the outlet 120 are not limited, but as illustrated in Fig. 3, it may be preferable that they be disposed at opposite locations.

The upper passage 111 is located at an upper side of the body 100 to communicate with the inlet 110, and preferably, an introduction pipe 111a connecting the inlet 110 and one side of the upper passage 111 such that the inlet 110 and the upper passage 111 communicate with each other. The upper passage 111 may be located in a circular groove shape, through which the fluid may flow, and a portion of the upper passage 111 may communicate with the inlet 110 through the introduction pipe 111a.

The lower passage 121 is located at a lower side of the body 100 to communicate with the outlet 120, and preferably, like the upper passage 111, the lower passage 121 may communicate with the outlet 120 through a discharge pipe 121 a connecting the outlet 120 and one side of the lower passage 121 such that the outlet 120 and the lower passage 121 communicate with each other, and to achieve this, auxiliary passages 121b may be located between the lower sides of the tube 200 and the lower passage 121.

The lower passage 121 may be located in a circular groove shape, through which the fluid may flow, and a portion of the lower passage 121 may communicate with the outlet 120 through the discharge pipe 121a, and it may be preferable that the lower passage 121 be parallel to the upper passage 111. That is, if it is assumed that the circumferences of the upper passage 111 and the lower passage 121 are connected to each other, a tubular shape having the shapes of the circumferences of the upper passage 111 and the lower passage 121 is made.

The accommodation part 130 is a specific space formed inside the body 100, and a plurality of accommodation parts 130 may be disposed to form a circumference that is smaller than the circumferences of the upper passage 111 and the lower passage 121. Although not illustrated, the accommodation parts 130 may be disposed to form a circumference that is larger than the circumferences of the upper passage 111 and the lower passage 121. That is, it is preferable that the upper passage 111 and the lower passage 121 be disposed in parallel to be spaced apart from each other at a specific interval.

Meanwhile, an indication part 140 is located on one side of the body 100, preferably, a side on which the inlet 110 is located, a location of the control lever 400 is determined according to the indication part 140 and a display part 410 of the control lever 400, which will be described below, which coincides with the display part 410 and it may be known.

The tubes 200 have hollow tubular shapes, and a plurality of tubes are accommodated in the interiors of the plurality of accommodation parts 130 and the accommodated tubes 200 have different inner diameters. The material of the tubes 200 is not limited, but preferably may be glass tubes 200.

As illustrated in Fig. 2, the sealing member includes a first sealing member 300 and a second sealing member 500.

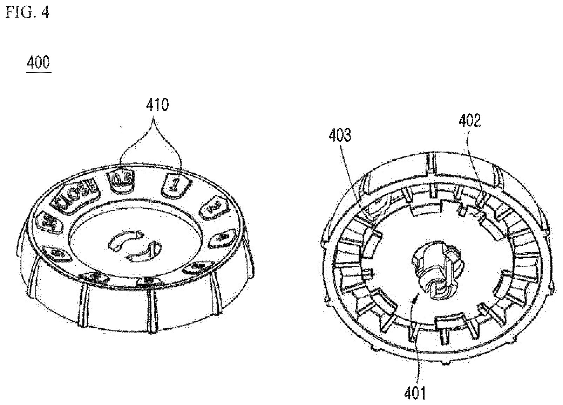

As illustrated in Figs. 2 and 4, the control lever 400 is located on the upper side of the body 100 to be rotatable. It may be preferable that a coupling part protruding from a lower surface of the body 100 is inserted into and coupled to a coupling hole 101 of the body 100 so that the control lever 400 is coupled to be rotatable, and the coupling part is formed of an elastic material to be inserted into the coupling hole 101 and to be pressed such that a diameter of the coupling part may be restored by an elastic force after being inserted after decreasing so that the body 100 may be coupled conveniently.

Further, a cover 420 in which display parts 410 that display a state of a flow rate of the fluid discharged through the outlet as a number are located and configured to cover an upper side of the control lever 400 may be provided on the upper side of the control lever 400. As described above, the rotation of the control lever 400 is adjusted by making any one of the display parts 410 coincide with the indication part 140 of the body 100.

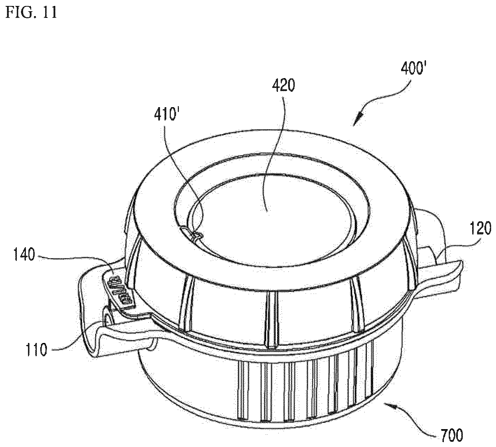

Further, as another embodiment of the control lever 400', as illustrated in Fig. 11, a display part in the form of numbers may not be displayed on the control lever 400', a state in which a display part 410' as a reference point simply indicating a closed state is located and the display part 410' coincides with the indication part 140 of the body 100 is a closed state, and a flow rate of the discharged fluid may be recognized through an angle between the display part 410' and the indication part 140 of the body 100.

More preferably, a fixing boss 403 is further located on one side of an inner peripheral surface of the control lever 400 and 400' and fixing recesses 103 are formed at locations corresponding to the accommodation parts 130 so that the fixing boss 403 may be accurately located at a location of a tube of the tubes 200 along an upper circumference of the body 100, which has a desired inner diameter, to be fixed as the control lever 400 rotates.

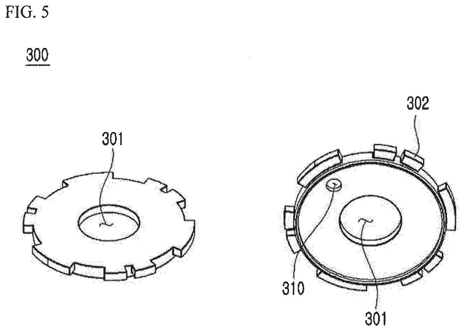

As illustrated in Figs. 2 and 5, the first sealing member 300 is interposed between the control lever 400 and the body 100 to cover the upper passage 111 and the upper sides of the tubes 200, on the upper side of the body 100, and to achieve this, preferably, the first sealing member 300 is located between the control lever 400 and the upper side of the body 100 by passing the coupling part 410 of the control lever 400 through a through-hole 301 formed at a central portion of the first sealing member 300 such that the coupling part 410 is coupled to the coupling hole 101 when the coupling part 410 is inserted into the coupling hole 101.

Further, the first sealing member 300 is disposed to be rotation in conjunction with the control lever 400. To achieve this, as illustrated in Figs. 3 and 4, a plurality of protrusions 302 are provided on an outer peripheral surface of the first sealing member 300 and recesses 402 corresponding to the number of the plurality of protrusions are located on a lower surface of the control lever 400 such that the first sealing member 300 is fixed to a lower side of the control lever 400 as the protrusions 302 and the recesses 402 are engaged with each other, and accordingly, the first sealing member 300 is rotated in conjunction with the first sealing member 300 as the control lever 400 is rotated.

Further, it may be preferable that the widths of the plurality of protrusions 302 of the first sealing member 300 be made to be different and the widths of the recesses 402 of the control lever 400 be made different to correspond to the protrusions 302 having different widths. Accordingly, an accurate location of a communication recess 310 may be coupled without any erroneous coupling when the first sealing member 300 is coupled to the control lever 400 by coupling the first sealing member 300 to the control lever 400 only at a predetermined location.

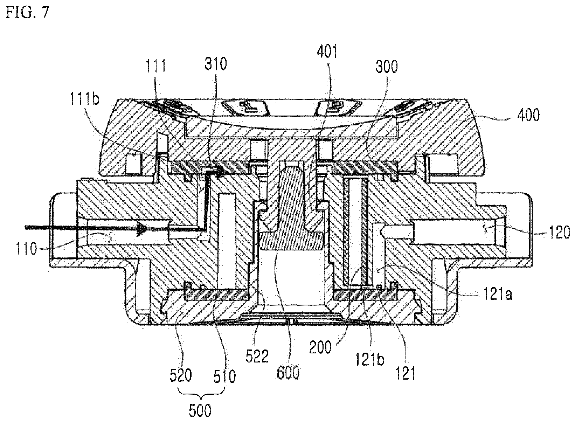

The communication recess 310, a portion of which is recessed, is located on a lower surface of the first sealing member 300. As illustrated in Figs. 5 and 7, the communication recess 310 is located such that the first sealing member 300 covers only the upper passage 111 (see the broken line of Fig. 5) or selectively covers the upper passage and the tubes 200 (see Fig. 7) as the first sealing member 300 rotates while the first sealing member 300 is located on the upper side of the body 100, and accordingly, the inlet 110 and the tubes 200 do not communicate with each other or the inlet 110 and the tubes 200 selectively communicate with each other according to a change of the location of the communication recess 310 due to rotation of the first sealing member 300.

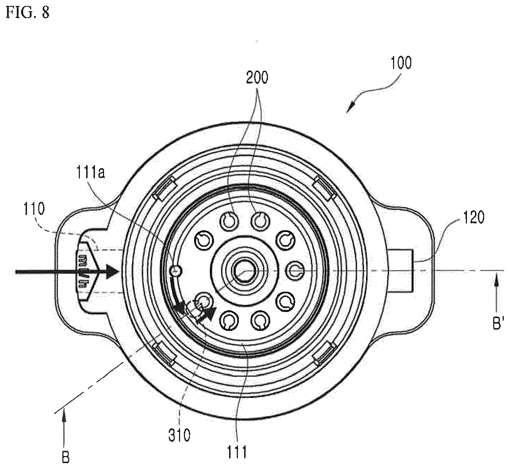

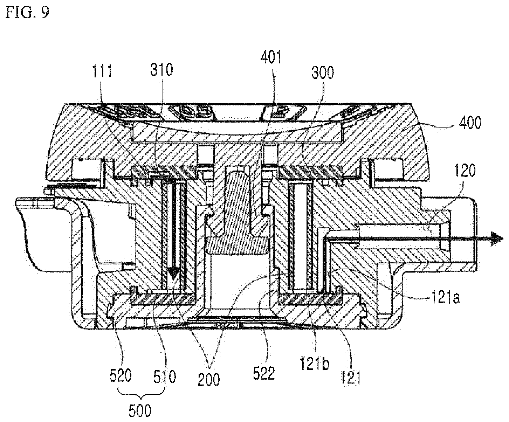

Further, as illustrated in Figs. 8 and 9, it is preferable that when the communication recess 310 is located to communicate some of the tubes 200, it overlap only a portion of the upper sides of the tubes 200. Accordingly, the lengthwise movements of the tubes 200 is restricted between the communication recess 310 and the second sealing member 500, which will be described below, when the fluid flows to the communicating tubes 200, and accordingly, when the tubes 200 are accommodated in the accommodation parts 130, the outer peripheral surfaces of the tubes 200 may be fixed without having to fix the outer peripheral surfaces of the tubes 200 to the inner peripheral surfaces of the accommodation parts 130 by using an adhesive.

Meanwhile, as illustrated in Fig. 2, the second sealing member 500 may include an inner sealing member 510 and an outer sealing member 520, and as illustrated in Figs. 2 and 7, the inner sealing member 510 is located to cover the lower passage 121 and the lower sides of the tubes 200 on the lower side of the body 100 to provide a watertight function to the lower passage 121.

The outer sealing member 520 is located on the lower side of the inner sealing member 510, and preferably, the coupling part 522 extending from a central portion to the upper side of the outer sealing member 520 is inserted into the coupling hole 101 of the body 100 to be coupled, and preferably, the coupling hole 521 is formed at a central portion of the coupling part 522 of the outer sealing member 520 such that the coupling part 410 of the control lever 400 may be inserted into and coupled to the coupling hole 521. That is, the control lever 400 and the outer sealing member 520 are coupled to each other while the body 100 is interposed between the control lever 400 and the outer sealing member 520, and more preferably, a pin 600 may pass through the coupling hole 521 of the outer sealing member 520 to be additionally coupled to the coupling part 410 of the control lever 400, and then, a stopper part 103 formed on the inner peripheral surface of the coupling hole 101 of the body 100 may be inserted into and fixed to a recess 523 formed on the outer peripheral surface of the coupling part 522 of the outer sealing member 520.

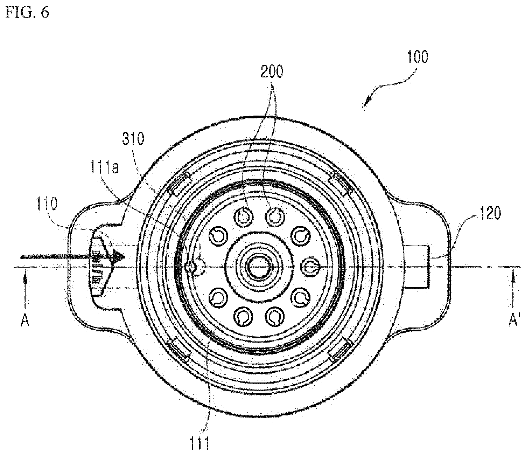

Further, the selector according to an embodiment of the present invention may further include a casing 700, and as illustrated in Figs. 2 and 6, the casing 700 is located to surround the outer side of the body 100 and a coupling step 702 is provided on the inner peripheral surface of the casing 700 such that the coupling step 702 may be coupled to an outside of the body 100 as the coupling step 702 is stopped by a stepped portion 102 formed on the outer peripheral surface of the body 100.

Due to the configuration of the selector according to the embodiment of the present invention, the inlet 110, the upper passage 111, the tube 200, the lower passage 121, and the outlet 120 may communicate with each other, and as described above, as the communication recess 310 is moved between the upper passage 111 and the tubes 200, the fluid may be introduced into the inlet 110 to be interrupted or may selectively flow through any one of the tubes 200 having different inner diameters so that the fluid may be interrupted or the flow rate of the fluid may be adjusted.

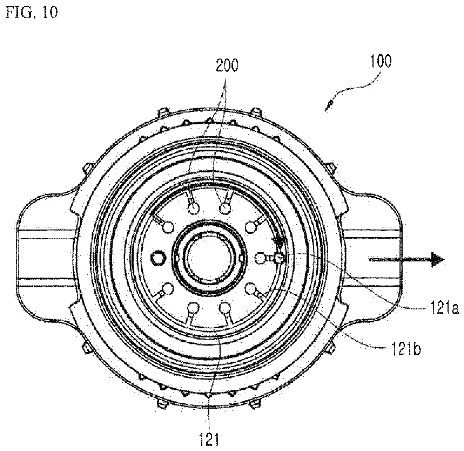

Hereinafter, an operation of the selector according to an embodiment of the present invention will be described in detail with reference to Figs. 6 to 10.

As illustrated in Figs. 6 and 7, in a state in which a closed state of the display part 410 of the control lever 400 coincides with the indication part as a state (hereinafter, a closed state) in which the communication recess 310 of the first sealing member 300 is located on a side on which the tubes 200 are not disposed, the flows of the fluid are interrupted so that the fluid cannot be discharged through the outlet 120 because the fluid introduced through the inlet 110 cannot communicate with the tubes 200 through the communication recess 310 (see the red arrows of Figs. 6 and 7).

Meanwhile, as illustrated in Figs. 8 to 10, in a state in which the communication recess 310 of the first sealing member 300 is located in any one of the tubes 200 disposed in the accommodation parts 130 of the body 100 through rotation of the control lever 400, that is, in a state other than the closed state, the fluid introduced through the inlet 110 flows into the communication recess 310 along the upper passage 111, flows to the tube 200 at which the communication recess 310 is located, and is discharged to the outlet 120 via the lower passage 121 because the upper passage 111 and any one of the tubes 200 communicate with each other through the communication recess 310 (see the red arrows of Figs. 8 to 10), and the communication recess 310 is located at a part at which the tube 200 having a desired inner diameter is located, by rotating the control lever 400 because the tubes 200 have different diameters as described above.

As described above, according to the selector of the present invention, the flowing fluid may be discharged at variously set quantities by interrupting or allowing the flows of the fluid through a simple operation of the control lever 400 so that the fluid may be accurately supplied consistently.

Further, because the configurations are assembled with each other through fitting, manufacturing costs may be reduced as the selector may be conveniently manufactured without using an adhesive.

Although the preferred embodiments of the present invention have been described, the present invention is not limited to the embodiments. That is, the present invention may be variously changed and corrected by those skilled in the art to which the present invention pertains, and all the modifications and equivalents have to be regarded as pertaining to the scope of the present invention.

## Claims

1. A selector comprising:
a body (100);
an inlet (110) located on one side of the body (100);
an outlet (120) located on the other side of the body (100);
a circular upper passage (111), one side of which communicates with the inlet (110) and which is located on an upper side of the body (100);
a circular lower passage (121), one side of which communicates with the outlet (120) and which is disposed in parallel to the lower side of the upper passage (111);
a plurality of tubes (200) disposed in parallel along circumferences of the upper passage (111) and the lower passage (121), having different inner diameters, and the lower sides of which communicate with the lower passage (121); and
a first sealing member (300) located to be rotatable to cover the upper side of the upper passage (111) and the upper sides of the plurality of tubes (200), on an upper side of the body (100) and having a communication recess (310) disposed to or not to overlap any one of the upper side of the upper passage (111) and the upper sides of the plurality of tubes (200), on a lower surface thereof,
wherein the upper passage (111) and the lower passage (121) selectively communicate with the tubes (200) according to a location of the communication recess (310) through rotation of the first sealing member (300),
wherein the inlet (110) and the tubes (200) do not communicate with each other or the inlet (110) and the tubes (200) selectively communicate with each other, according to a change of the location of the communication recess (310) due to rotation of the first sealing member (300).

2. The selector of claim 1, further comprising:
a control lever (400, 400') located on the upper side of the body (100),
wherein the first sealing member (300) is located on one side of the control lever (400, 400') to be rotated in conjunction with the control lever (400, 400') as the control lever (400, 400') is rotated.

3. The selector of claim 2, wherein the first sealing member (300) includes a plurality of protrusions (302) protruding from an outer peripheral surface of the first sealing member (300),
wherein the control lever (400, 400') includes recesses (402) formed on one side of an inner peripheral surface of the control lever (400, 400') and the number of which is the same as that of the protrusions (302), and
wherein the first sealing member (300) is rotated in conjunction with the first sealing member (300) as the control lever (400, 400') is rotated as the plurality of protrusions (302) is engaged with the recesses (402) to be fixed.

4. The selector of claim 3, wherein the widths of the plurality of protrusions are different, and the widths of the recesses (402) correspond to the widths of the plurality of protrusions (302).

5. The selector of any one of claims 2 to 4, further comprising:
a display part (410) located on one side of the control lever (400, 400').

6. The selector of any one of the preceding claims, further comprising:
a second sealing member (500) located on a lower side of the body (100) and covering the tubes (200) and the lower passage (121).

7. The selector of claim 6, wherein movement of the tubes (200) in lengthwise directions thereof is restricted by vertically locating the tubes (200) between the first sealing member (300) and the second sealing member (500).

8. The selector of any one of claims 2 to 7, wherein a fixing boss (403) is further provided on one side of an inner peripheral surface of the control lever (400, 400'),
wherein the body (100) includes a plurality of fixing recesses (103) formed at locations corresponding to the tubes (200) along an upper circumference of the body (100), and
wherein the fixing boss (403) is inserted into any one of the plurality of fixing recesses (103) as the control lever (400, 400') is rotated.

## Patentansprüche

1. Wählschalter, umfassend:
einen Körper (100);
einen Einlass (110), der sich auf einer Seite des Körpers (100) befindet;
einen Auslass (120), der sich auf der anderen Seite des Körpers (100) befindet;
einen kreisförmigen oberen Abschnitt (111), dessen eine Seite mit dem Einlass (110) in Verbindung steht und der sich auf einer Oberseite des Körpers (100) befindet;
einen kreisförmigen unteren Abschnitt (121), dessen eine Seite mit dem Auslass in Verbindung steht (120) und der parallel zur Unterseite des oberen Abschnitts (111) angeordnet ist;
eine Vielzahl von Rohren (200), die parallel entlang des Umfangs des oberen Abschnitts (111) und dem unteren Abschnitt (121) angeordnet sind, die unterschiedliche Innendurchmesser haben und deren Unterseiten mit dem unteren Abschnitt (121) in Verbindung stehen; und
ein erstes Dichtungselement (300), das drehbar angeordnet ist, um mit der Oberseite des oberen Abschnitts (111) und die Oberseiten der Vielzahl von Rohren (200) auf einer Oberseite des Körpers (100) zu überlappen oder nicht, und eine Verbindungsaussparung (310) aufweist, die so angeordnet ist, dass sie entweder mit der Oberseite des oberen Abschnitts (111) oder den Oberseiten der Vielzahl von Rohren (200) auf einer Unterseite davon überlappt oder nicht, wobei der obere Abschnitt (111) und der untere Abschnitt (121) wahlweise mit den Rohren (200) entsprechend einer Stelle der Verbindungsaussparung (310) durch Drehung des ersten Dichtungselements (300) in Verbindung stehen, wobei der Einlass (110) und die Rohre (200) nicht miteinander in Verbindung stehen oder der Einlass (110) und die Rohre (200) wahlweise miteinander in Verbindung stehen, entsprechend einer Änderung der Stelle der Verbindungsaussparung (310) aufgrund der Drehung des ersten Dichtungselements (300).

2. Wählschalter nach Anspruch 1, ferner umfassend:
einen Steuerhebel (400, 400'), der auf der Oberseite des Körpers (100) angeordnet ist, wobei das erste Dichtungselement (300) auf einer Seite des Steuerhebels (400, 400') angeordnet ist, um in Übereinstimmung mit dem Steuerhebel (400, 400') gedreht zu werden, wenn der Steuerhebel (400, 400') gedreht wird.

3. Wählschalter nach Anspruch 2, wobei
das erste Dichtungselement (300) eine Vielzahl von Vorsprüngen (302), die von einer äußeren Umfangsfläche des ersten Dichtungselements (300) vorstehen,
wobei der Steuerhebel (400, 400') Aussparungen (402) aufweist, die auf einer Seite einer inneren Umfangsfläche des Steuerhebels (400, 400') ausgebildet sind und deren Anzahl die gleiche ist wie die der Vorsprünge (302), und
wobei das erste Dichtungselement (300) in Übereinstimmung mit dem ersten Dichtungselement (300) gedreht wird, wenn der Steuerhebel (400, 400') gedreht wird, wenn die Vielzahl von Vorsprüngen (302) mit den zu befestigenden Aussparungen (402) in Eingriff gebracht wird.

4. Wähl schalter nach Anspruch 3, wobei
die Breiten der mehreren Vorsprünge unterschiedlich sind und die Breiten der Ausnehmungen (402) den Breiten der mehreren Vorsprünge (302) entsprechen.

5. Wählschalter nach einem der Ansprüche 2 bis 4, ferner umfassend:
ein Anzeigeteil (410), das sich auf einer Seite des Steuerhebels (400, 400') befindet.

6. Wählschalter nach einem der vorhergehenden Ansprüche, ferner umfassend:
ein zweites Dichtungselement (500), das sich an einer Unterseite des Körpers (100) befindet und die Rohre (200) und den unteren Abschnitt (121) abdeckt.

7. Wähl schalter nach Anspruch 6, wobei
die Bewegung der Rohre (200) in deren Längsrichtung durch eine vertikale Anordnung der Rohre (200) zwischen dem ersten Dichtungselement (300) und dem zweiten Dichtungselement (500) begrenzt ist.

8. Wähl schalter nach einem der Ansprüche 2 bis 7 wobei
auf einer Seite einer inneren Umfangsfläche des Steuerhebels (400, 400') ferner ein Befestigungsvorsprung (403) vorgesehen ist,
wobei der Körper (100) eine Vielzahl von Befestigungsaussparungen (103) aufweist, die an Stellen ausgebildet sind, die den Rohren (200) entlang eines oberen Umfangs des Körpers (100) entsprechen, und
wobei der Befestigungsvorsprung (403) in eine beliebige der mehreren Befestigungsaussparungen (103) eingeführt wird, wenn der Steuerhebel (400, 400') gedreht wird.

## Revendications

1. Sélecteur comprenant :
un corps (100) ;
une entrée (110) située sur un côté du corps (100) ;
une sortie (120) située sur l'autre côté du corps (100) ;
un passage supérieur circulaire (111) dont un côté communique avec l'entrée (110) et qui est situé sur un côté supérieur du corps (100) ;
un passage inférieur circulaire (121) dont un côté communique avec la sortie (120) et qui est disposé parallèlement au côté inférieur du passage supérieur (111) ;
une pluralité de tubes (200) disposés en parallèle le long de circonférences du passage supérieur (111) et du passage inférieur (121), présentant des diamètres intérieurs différents, et dont les côtés inférieurs communiquent avec le passage inférieur (121) ; et
un premier organe d'étanchéité (300) mis en place pour pouvoir tourner afin de recouvrir le côté supérieur du passage supérieur (111) et les côtés supérieurs de la pluralité de tubes (200), sur un côté supérieur du corps (100) et présentant un évidement de communication (310) disposé pour chevaucher ou non l'un quelconque du côté supérieur du passage supérieur (111) et des côtés supérieurs de la pluralité de tubes (200), sur une surface inférieure de celui-ci,
dans lequel le passage supérieur (111) et le passage inférieur (121) communiquent de manière sélective avec les tubes (200) en fonction d'un emplacement de l'évidement de communication (310) par rotation du premier organe d'étanchéité (300),
dans lequel l'entrée (110) et les tubes (200) ne communiquent pas entre eux ou l'entrée (110) et les tubes (200) communiquent entre eux de manière sélective, en fonction d'une modification de l'emplacement de l'évidement de communication (310) due à la rotation du premier organe d'étanchéité (300).

2. Sélecteur selon la revendication 1, comprenant en outre :
une manette de commande (400, 400') située sur le côté supérieur du corps (100),
dans lequel le premier organe d'étanchéité (300) est situé sur un côté de la manette de commande (400, 400') pour être tourné conjointement avec la manette de commande (400, 400'), à mesure que la manette de commande (400, 400') est tournée.

3. Sélecteur selon la revendication 2, dans lequel le premier organe d'étanchéité (300) présente une pluralité de saillies (302) faisant saillie depuis une surface périphérique extérieure du premier organe d'étanchéité (300),
dans lequel la manette de commande (400, 400') présente des évidements (402) formés sur un côté d'une surface périphérique intérieure de la manette de commande (400, 400') et dont le nombre est identique à celui des saillies (302), et
dans lequel le premier organe d'étanchéité (300) est tourné conjointement avec le premier organe d'étanchéité (300) à mesure que la manette de commande (400, 400') est tournée, tandis que la pluralité de saillies (302) se loge dans les évidements (402), pour être fixé.

4. Sélecteur selon la revendication 3, dans lequel les largeurs de la pluralité de saillies sont différentes, et les largeurs des évidements (402) correspondent aux largeurs de la pluralité de saillies (302).

5. Sélecteur selon l'une quelconque des revendications 2 à 4, comprenant en outre :
une partie d'affichage (410) située sur un côté de la manette de commande (400, 400').

6. Sélecteur selon l'une quelconque des revendications précédentes, comprenant en outre :
un second organe d'étanchéité (500) situé sur un côté inférieur du corps (100) et recouvrant les tubes (200) ainsi que le passage inférieur (121).

7. Sélecteur selon la revendication 6, dans lequel le mouvement des tubes (200) dans les directions longitudinales de ceux-ci est limité par mise en place verticale des tubes (200) entre le premier organe d'étanchéité (300) et le second organe d'étanchéité (500).

8. Sélecteur selon l'une quelconque des revendications 2 à 7, dans lequel un bossage de fixation (403) se trouve en outre sur un côté de surface périphérique intérieure de la manette de commande (400, 400'),
dans lequel le corps (100) présente une pluralité d'évidements de fixation (103) formés à des emplacements correspondant aux tubes (200) le long d'une circonférence supérieure du corps (100), et
dans lequel le bossage de fixation (403) s'insère dans un évidement quelconque de la pluralité d'évidements de fixation (103), à mesure que la manette de commande (400, 400') est tournée.
